# FASCICULE DE BREVET EUROPEEN

(11) **EP 0 888 109 B1**
(45) Date de publication et mention de la délivrance du brevet: **30.08.2000**
(21) Numéro de dépôt: 97915538.9
(22) Date de dépôt: 21.03.1997
(51) Int. Cl.: A61K 7/13

(54) **COMPOSITION DE TEINTURE DES FIBRES KERATINIQUES CONTENANT DES PYRAZOLO-PYRIMIDINEOXO; LEUR UTILISATION POUR LA TEINTURE COMME COUPLEURS, PROCEDES DE TEINTURE**
ZUSAMMENSETZUNG ZUR FÄRBUNG VON KERATINISCHEN FASERN ENTHALTEND PYRAZOLOOXOPYRIMIDIN ALS KUPPLER UND FÄRBEVERFAHREN
KERATIN FIBRE DYE COMPOSITION CONTAINING PYRAZOLO-PYRIMIDINEOXO COMPOUNDS, USE THEREOF AS DYE COUPLERS, AND DYEING METHODS

(30) Priorité: 22.03.1996 FR 9603629
(43) Date de publication de la demande: 07.01.1999
(73) Titulaire: L'OREAL, 75008 Paris (FR)
(72) Inventeur: VIDAL, Laurent, F-75013 Paris (FR); MALLE, Gérard, 77580 Villiers S/Morin (FR)
(74) Mandataire: Goulard, Sophie
(86) Numéro de dépôt international: FR9700506
(87) Numéro de publication internationale: WO9735550

(56) Documents cités:
- EP-A- 0 030 680
- WO-A-92/04883
- DE-A- 4 133 957

## Description

L'invention a pour objet une composition pour la teinture par oxydation des fibres kératiniques en particulier des cheveux humains contenant au moins un composé pyrazolo-pyrimidineoxo comme coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs et être enfin les moins sélectifs possible, c'est-à-dire permettre des écarts de coloration les plus faibles possible tout au long d'une même fibre kératinique, qui peut être en effet, différemment sensibilisée (i.e. abîmée) entre sa pointe et sa racine.

Il a déjà été proposé, notamment dans la demande de brevet WO 92/04883 d'utiliser à titre de coupleur, des pyrazolo-(1,5-a)-pyrimidines comportant obligatoirement une fonction hydroxyle sur le cycle pyrazole.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des composés pyrazolo-pyrimidineoxo comme coupleurs en présence d'une base d'oxydation.

Cette découverte est à la base de la présente invention.

L'invention a pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un composé pyrazolo-pyrimidineoxo de formule (I) ou l'un de ses sels d'addition avec un acide : 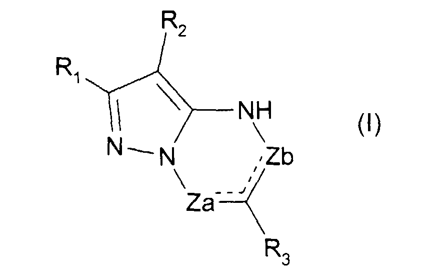 dans laquelle :
   . R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), éventuellement substitué par 1 ou 2 radicaux R tels que définis précédemment ;
      lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
      R₁ peut désigner aussi un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy ; un radical amino ; un radical acrylamino ; un radical acyloxy ; un radical carbamoyloxy ; un radical sulfonamide ; un radical imide ; un radical uréido ; un radical sulfamoylamino ; un radical alcoxy carbonylamino ; un radical aryloxycarbonylamino ; un radical alcoxycarbonyle ; un radical aryloxycarbonyle ; un radical carboxyle.
   . R₂ représente : un atome d'hydrogène ; un atome d'halogène tel que brome, chlore ou fluor ; un groupe acétylamido ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chlorobenzyloxy, méthoxy éthylcarbamoylméthoxy) ; un radical aryloxy (tel que par exemple : phénoxy, 4-méthoxyphénoxy, 4-nitrophénoxy, 4-cyanophénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristyloxy) ; un radical arylthio (tel que par exemple : phénylthio, 4-carboxyphénylthio, 4-méthanesulfonyphénylthio) ; un radical alkylthio (tel que par exemple : méthylthio, éthylthio, propylthio, butylthio, 2-cyanoéthylthio, benzylthio, phénéthylthio, 2-(diéthylamino)éthylthio, éthoxyéthylthio, phénoxyéthylthio) ; un radical hétéroarylthio (tel que par exemple : 5-phényl 2,3,4,5-tétrazolylthio, 2-benzothiazolylthio) ; un radical hétéroaryloxy (tel que par exemple : 5-phényl 2,3,4,5-tétrazolyloxy, 2-benzothiazolyloxy) ; un radical thiocyano ; un radical N,N-diéthyl thiocarbonylthio ; un radical dodécyloxythiocarbonylthio ; un radical benzènesulfanomido ; un radical N-éthyltoluène sulfonamido ; un radical pentafluorobutanamido ; un radical 2,3,4,5,6-pentafluorobenzamido ; un radical p-cyanophényluréido, un radical N,N-diéthylsulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle; 5,5-diméthyl 2,4-dioxo 3-oxazolidinyle ; un radical 2-oxy 1,2-dihydro 1-pyridinyle ; un alkylamido ; un arylamido ; un radical NR^{lll}R^{lV} avec R^{lll} et R^{lV} représentant, identiques ou différents, un alkyle en C₁-C₄, un hydroxyalkyle ; un carboxyle ; ou un radical alcoxycarboxylique.
      R₃ a les mêmes significations indiquées que celles indiquées pour le radical R₁. Zₐ et Z_{b} sont différents et représentent un groupe C=O ou un atome de carbone portant un radical R₄ ayant les mêmes significations que celles indiquées pour le radical R₁ ;
   . et au moins une base d'oxydation.

Les sels d'addition avec un acide des composés de l'invention peuvent être choisis notamment parmi des chlorhydrates, les bromhydrates, les tartrates, les tosylates, les benzènesulfonates, les sulfates, les lactates et les acétates.

Parmi les radicaux R₁ de la formule (I) définie ci-dessus, on préfère les radicaux choisis dans le groupe constitué par :
un atome d'hydrogène ; un alkyle en C₁-C₄, linéaire ou ramifié ; un phényle ; un phényle substitué par un atome d'halogène, un alkyle en C₁-C₄, un alcoxy en C₁-C₄, un groupe nitro, un groupe amino, un groupe trifluorométhyle ou alkylamino en C₁-C₄ ; un radical benzyle ; un radical benzyle substitué par un atome d'halogène, un alkyle en C₁-C₄, un alcoxy en C₁-C₄, un groupe nitro, un groupe amino, un groupe trifluorométhyle ; un alkylamino en C₁-C₄ ; un hétérocycle choisi parmi le thiophène, le furane ou la pyridine ; un radical trifluorométhyle ; un radical (CH₂)ₚ-X-(CH₂)_{q}-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C₄ ; un alkylamino en C₁-C₄ ; un dialkylamino en C₁-C₄ ; un phényloxycarbonyle ; méthylthio ; éthylthio ; phénylthio ; méthanesulfonyle ; cyano ; un arylamino ; un radical alcoxy choisi parmi méthoxy, éthoxy, phénoxy ; un halogène choisi parmi chlore, brome, fluor ; un groupe carboxyle ; un alcoxycarbonyle en C₁-C₄.

Parmi les radicaux R₁ de la formule (I) définie ci-dessus, on préfère plus particulièrement les radicaux choisis dans le groupe constitué par :
hydrogène ; un alkyle choisi parmi méthyle, éthyle, isopropyle, ter-butyle ; un halogène choisi parmi fluor et chlore ; phényle ; toluyle ; 4-chlorophényle ; 4-méthoxyphényle ; 3-méthoxyphényle ; 2-méthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

Et encore plus particulièrement, on préfère les radicaux R₁ choisis dans le groupe constitué par :
hydrogène ; méthyle ; éthyle ; phényle ; toluyle ; 4-chlorophényle ; 4-méthoxyphényle ; benzyle ; trifluorométhyle ; chloro ; un radical méthoxy ou éthoxy ; un radical carboxyle ; méthylamino ou diméthylamino ; cyano.

Parmi les radicaux R₂ de la formule (I) définie ci-dessus, on préfère les radicaux choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{lll}R^{lV} avec R^{lll} et R^{lV} représentant, identiques ou différents, un alkyle en C₁-C₄, un hydroxyalkyle en C₁-C₄ ; un carboxyle ; un radical alcoxycarboxylique en C₁-C₄.

Parmi les radicaux R₂ de la formule (I) définie ci-dessus, on préfère plus particulièrement les radicaux choisis dans le groupe constitué par :
hydrogène ; chlore ou brome ; méthoxy ou éthoxy ; phénoxy ; 4-méthylphénoxy ; acyloxy ; benzyloxy ; méthylthio ou éthylthio ; phénylthio ; 4-méthylphénylthio ; 2-tertiobutylphénylthio ; acétamido ; phénylacétamido ; diméthylamino ; diéthylamino ; éthylméthylamino ; (β-hydroxyéthyl) méthylamino.

Et encore plus particulièrement, on préfère les radicaux R₂ choisis dans le groupe constitué par : hydrogène ; chlore ; éthoxy ; phénoxy ; benzyloxy ; acyloxy ; acétamido : diméthylamino.

Parmi les radicaux R₃ et R₄ de la formule (I) définie ci-dessus, on préfère les radicaux choisis dans le groupe constitué par :
un atome d'hydrogène ; un alkyle en C₁-C₄, linéaire ou ramifié, éventuellement substitué par un hydroxy ou amino ; un phényle ; un phényle substitué par un ou deux groupes choisis parmi un halogène, un alkyle en C₁-C₄, un alcoxy en C₁-C₄ ; un hydroxy ; un carboxyle, un groupe nitro, un alkylthio en C₁-C₄, un groupe méthylènedioxy, un groupe amino, un groupe trifluorométhyle ou un alkylamino en C₁-C₄ ; un radical benzyle ; un radical benzyle substitué par un atome d'halogène, un méthyle ou isopropyle, méthoxy ; un hydroxyalkyle en C₁-C₄ ; un aminoalkyle en C₁-C₄ ; un alkylaminoalkyle en C₁-C₄ ; un radical amino ; un radical alkylamino en C₁-C₄, un halogène tel que chlore ou brome ; un trifluorométhyle.

Parmi les radicaux R₃ et R₄, on préfère les radicaux choisis dans le groupe constitué par : hydrogène ; alkyle en C₁-C₄ (tel que méthyle ; éthyle ; isopropyle) ; halogène (tel que chlore, brome) ; amino ; alkylamino en C₁-C₄ : tel que méthylamino, éthylamino ou diméthylamino ; un radical aryle (tel que phényle, toluyle, 2-, 3- ou 4-chloro-phényle, 3- ou 4-hydroxyphényle, 3- ou 4-aminophényle, 3- ou 4-méthoxyphényle, 4-trifluoro-méthylphényle) ; benzyle ; hydroxyméthyle ou hydroxyéthyle ; aminométhyle ou aminoéthyle ; trifluorométhyle.

Et encore plus particulièrement, on préfère les radicaux R₃ et R₄ choisis parmi les radicaux suivants :
hydrogène, méthyle, éthyle, isopropyle, trifluorométhyle, chlore, amino, méthylamino, éthylamino, phényle, 4-chlorophényle, 4-méthoxyphényle.

Parmi les composés de l'invention de formule (I) préférentiels, on peut citer ceux choisis dans le groupe constitué par :
(i) les pyrazolo-[1,5-a]-pyrimidine-7-oxo de formule : 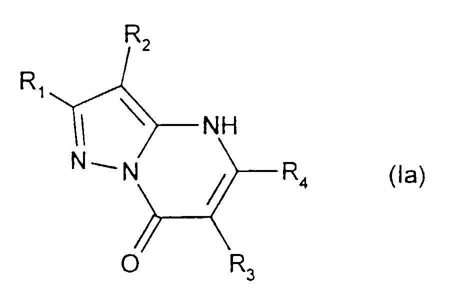
(ii) les pyrazolo-[1,5-a]-pyrimidine-5-oxo de formule : 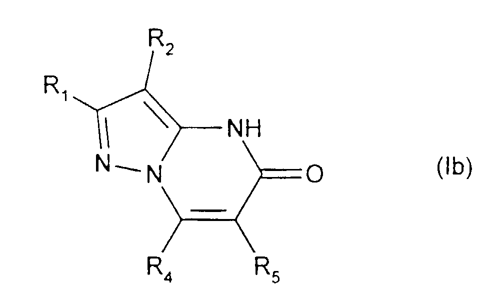 dans lesquelles les radicaux R₁, R₂, R₃ et R₄ ont les mêmes significations que celles indiquées ci-dessus.

Comme exemples de composés de formule (la) on peut citer ceux pour lesquels :
R₁ désigne hydrogène, méthyle, éthyle, chlore, phényle, méthoxy, trifluorométhyle, carboxyle ou cyano ;
R₂ désigne hydrogène, chlore ou éthoxy ;
R₃ et R₄ désignent respectivement : hydrogène et hydrogène ; hydrogène et méthyle ; méthyle et hydrogène ; hydrogène et amino ; chlore et méthyle ; chlore et amino ; carboxyle et méthyle ; hydrogène et trifluorométhyle ou carboxyle et hydrogène.

A titre de composés de formule (la) ci-dessus, on peut tout particulièrement citer :
- le pyrazolo [1,5-a] pyrimidin-7-one,
- le 2-méthyl pyrazolo [1,5-a] pyrimidin-7-one,
- le 2-phényl pyrazolo [1,5-a] pyrimidin-7-one,
- le 2-carboxy pyrazolo [1,5-a] pyrimidin-7-one,
- le 2-éthylthio pyrazolo [1,5-a] pyrimidin-7-one,
- le 2,5-diméthyl pyrazolo [1,5-a] pyrimidin-7-one,
- le 2-phényl-5-méthyl pyrazolo [1,5-a] pyrimidin-7-one,
- le 2-carboxy-5-méthyl pyrazolo [1,5-a] pyrimidin-7-one,
- le 2-éthylthio-5-méthylpyrazolo [1,5-a] pyrimidin-7-one,
- le 2-méthyl-5-trifluorométhyl pyrazolo [1,5-a] pyrimidin-7-one,
- le 2-phényl-5-trifluorométhyl pyrazolo [1,5-a] pyrimidin-7-one,
- le 2-carboxy-5-trifluorométhyl pyrazolo [1,5-a] pyrimidin-7-one,
- le 2-éthylthio-5-trifluorométhyl pyrazolo [1,5-a] pyrimidin-7-one,
- le 5-trifluorométhyl pyrazolo [1,5-a] pyrimidin-7-one,
- le 5-méthyl pyrazolo [1,5-a] pyrimidin-7-one,
- le 6-carboxy pyrazolo [1,5-a] pyrimidin-7-one,
- le 6-carboxy-2-méthyl pyrazolo [1,5-a] pyrimidin-7-one,
- le 6-carboxy-2-phényl pyrazolo [1,5-a] pyrimidin-7-one,
- le 6-carboxy-2-éthylthio pyrazolo [1,5-a] pyrimidin-7-one,
- le 2,6-dicarboxy pyrazolo [1,5-a] pyrimidin-7-one,
- le 2-méthyl-6-éthoxycarbonyl pyrazolo [1,5-a] pyrimidin-7-one,
- le 2-(2'-furyl)-6-méthyl pyrazolo [1,5-a] pyrimidin-7-one,
- le 2-(2'-thienyl)-6méthyl pyrazolo [1,5-a] pyrimidin-7-one,
- le 3-éthoxycarbonyl-6-méthyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
et leurs sels d'addition avec un acide.

Comme exemples de composés de formule (Ib), on peut citer ceux pour lesquels R₁, R₂, R₃ et R₄ ont les mêmes significations que celles indiquées dans les exemples de composés de formule (la) définis ci-dessus.

A titre de composés de formule (Ib) ci-dessus, on peut tout particulièrement citer :
- le pyrazolo [1,5-a] pyrimidin-5-one,
- le 2-méthyl pyrazolo [1,5-a] pyrimidin-5-one,
- 2,7-diméthyl pyrazolo [1,5-a] pyrimidin-5-one,
- le 2-phényl pyrazolo [1,5-a] pyrimidin-5-one,
- le 2-éthylthio pyrazolo [1,5-a] pyrimidin-5-one,
- le 2-carboxy pyrazolo [1,5-a] pyrimidin-5-one,
- le 7-amino pyrazolo [1,5-a] pyrimidin-5-one,
- le 7-amino-2-méthyl pyrazolo [1,5-a] pyrimidin-5-one,
- le 7-amino-2-phényl pyrazolo [1,5-a] pyrimidin-5-one,
- le 7-amino--2-éthylthio pyrazolo [1,5-a] pyrimidin-5-one,
- le 7-amino-2-carboxy pyrazolo [1,5-a] pyrimidin-5-one,
et leurs sels d'addition avec un acide.

Les composés de la présente invention, et leurs procédés sont décrits dans la demande de brevet EP-A-304001.

Leurs intermédiaires de synthèse sont décrits dans les demandes de brevet EP-A-591 103, WO 92/04349, EP-A-320 764 et dans les publications suivantes :
- C. MUSANTE, Gazetta Chim. ltal. 73, 355, 1943 ;
- H. DORN, Liebigs Ann. Chem. 707,141,1967 ;
- H. DORN, Liebigs Ann. Chem. 717, 118, 1968 ;
- P. ARNOLD, Angew. Chem. Int. ed., 13, 206, 1974 ;
- K. TAKAHASHI, Synthesis, 794, 1985 ;
- C.B. VICENTINI, il Farmaco, 47, (7,8), 1021, 1992 ;
- K.S. HARTKE, J. Am. Chem. Soc., 81, 2456, 1959 ;
- C.B. VICENTINI, J. Het. Chem., 26, 797, 1989.

Le ou les composés de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante : 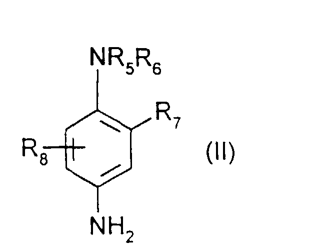 dans laquelle :
R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄) ;
R₆ représente un atome d'hydrogène, un radical alkyle en C₁-C₄, monohydroxyalkyle en C₁-C₄ ou polyhydroxyalkyle en C₂-C₄,
R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₈ représente un atome d'hydrogène ou un radical alkyle en C₁-C₄.

Dans la formule (II) ci-dessus, et lorsque R₇ est différent d'un atome d'hydrogène, alors R₅ et R₆ représentent de préférence un atome d'hydrogène et R₇ est de préférence identique à R₈, et lorsque R₇ représente un atome d'halogène, alors R₅, R₆ et R₈ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylènediamine, la 2-β-hydroxyéthyloxy paraphénylènediamine, la 2,6-diméthyl paraphénylènediamine, la 2,6-diéthyl paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 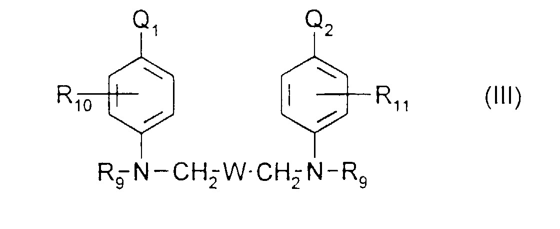 dans laquelle
Q₁ et Q₂, identiques ou différents, représentent un radical hydroxyle ou NHR₁₂ dans lequel R₁₂ représente un atome d'hydrogène ou un radical alkyle en C₁-C₄,
R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₀ et R₁₁, identiques ou différents, représentent un atome d'hydrogène ou d'halogène ou un radical alkyle en C₁-C₄,
W représente un radical pris dans le groupe constitué par les radicaux suivants :
   -(CH₂)ₙ ; -(CH₂)ₘ-O-(CH₂)ₘ ; -(CH₂)ₘ-CHOH-(CH₂)ₘ et 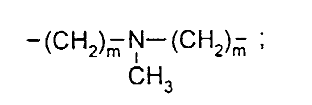 dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les paraaminophénols utilisables à titre de bases d'oxydation dans la composition tinctoriale selon l'invention, on peut notamment citer les composés répondant à la formule (IV) suivante, et leurs sels d'addition avec un acide : 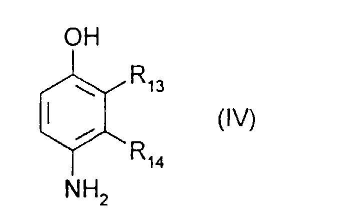 dans laquelle :
R₁₃ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétraaminopyrimidine, la 4-hydroxy 2,5,6-triamino-pyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole et le 1-(4'-chlorobenzyl)-4,5-diaminopyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'a-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents, ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 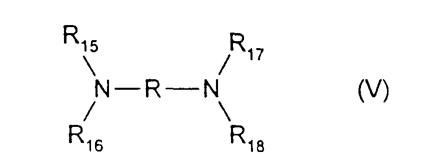 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des pyrazolo-pyrimidineoxo de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. II est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus.

Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES

### EXEMPLES 1 à 3 DE TEINTURE EN MILIEU ALCALIN

| **Exemples** | **1** | **2** | **3** |
|---|---|---|---|
| 2,7-diméthyl pyrazolo [1,5-a] pyrimidin-5-one (coupleur) | 0,490 | 0,490 | - |
| 2,5-diméthyl pyrazolo [1,5-a] pyrimidin-7-one (coupleur) | - | - | 0,490 |
| Paraphénylènediamine (base d'oxydation) | 0,324 | 0,324 | 0,324 |
| Support de teinture commun | n°1 | n°1 | n°1 |
| Eau déminéralisée q.s.p. | 100 g | 100 g | 100 g |
| **NB** : Le 2,7-diméthyl pyrazolo [1,5-a] pyrimidin-5-one a été préparé selon le procédé décrit dans la demande de brevet EP-A-304 001, et le 2,5-diméthyl pyrazolo [1,5-a] pyrimidin-7-one est commercialisé sous la dénomination commerciale KM 00085 par la société MAYBRIDG | | | |

### Support de teinture commun n°1 :

- Alcool benzylique 2,0 g
- Polyéthylène glycol à 6 moles d'oxyde d'éthylène 3,0 g
- Ethanol 20,0 g
- Alkyl (C₈-C₁₀) polyglucoside en solution aqueuse à 60% de matière active, tamponné par du citrate d'ammonium, vendu sous la dénomination ORAMIX CG110 par la société SEPPIC 6,0 g
- Ammoniaque à 20% de NH₃ 10,0 g
- Métabisulfite de sodium 0,228 g
- Agent séquestrant q.s.

Au moment de l'emploi, la composition tinctoriale de l'exemple 1 ci-dessus a été mélangée avec un poids égal d'une solution de peroxyde d'hydrogène à 20 volumes (6% en poids) ; chacune des compositions tinctoriales des exemples 2 et 3 ci-dessus a été mélangée avec un poids égal d'une solution aqueuse de persulfate d'ammonium à 6.10⁻³ mole %.

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 1 ci-dessous :

**TABLEAU 1**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **1** | 9,9 | Irisé | Irisé légèrement rouge |
| **2** | 10,1 | Irisé | Irisé rouge puissant |
| **3** | 9,8 | Blond très clair légèrement doré | Blond très clair légèrement doré |

### EXEMPLE 4 DE TEINTURE EN MILIEU NEUTRE

On a préparé la composition tinctoriale conforme à l'invention suivante :
- 2,5-diméthyl pyrazolo [1,5-a] pyrimidin-7-one 0,490 g
- Paraphénylènediamine 0,324 g
- Alcool benzylique 2,0 g
- Polyéthylène glycol 6 OE 3,0 g
- Ethanol 20,0 g
- Alkyl (C₈-C₁₀) polyglucoside en solution aqueuse à 60% de matière active tamponné par du citrate d'ammonium vendu sous la dénomination ORAMIX CG110 par la société SEPPIC 6,0 g
- Tampon K₂HPO₄ / KH₂PO₄ (1.5 M / 1 M) 10,0 g
- Métabisulfite de sodium 0,228 g
- Agent séquestrant q.s.

Au moment de l'emploi, la composition tinctoriale de l'exemple 4 ci-dessus a été mélangée avec un poids égal d'une solution aqueuse de persulfate d'ammonium à 6.10⁻³ mole %.

Le mélange obtenu présentait un pH de 7,2 et a été appliqué pendant 30 minutes, sur des mèches de cheveux gris naturels à 90 % de blancs, à raison de 10 g pour 1 g de cheveux. Après rinçage, lavage avec un shampooing standard et séchage, les mèches ont été teintes dans une nuance Blond très clair légèrement doré.

### EXEMPLES 5 à 7 DE TEINTURE EN MILIEU ALCALIN

| **Exemples** | **5** | **6** | **7** |
|---|---|---|---|
| 2-méthyl-5-méthoxyméthyl pyrazolo [1,5-a] pyrimidin-7-one (coupleur) | 0,579 | - | - |
| 2-ter-butyl-5-trifluorométhyl pyrazolo [1,5-a] pyrimidin-7-one (coupleur) | - | 0,777 | - |
| 2-méthyl-6-éthoxycarbonyl pyrazolo [1,5-a] pyrimidin-7-one (coupleur) | - | - | 0,663 |
| Dichlorhydrate de 1,3-diméthyl-4,5-diamino pyrazole (base d'oxydation) | 0,597 | - | 0,597 |
| Dichlorhydrate de N,N-bis-(β-hydroxyéthyl) paraphénylènediamine (base d'oxydation) | - | 0,807 | - |
| Support de teinture commun | n°1 | n°1 | n°1 |
| Eau déminéralisée q.s.p. | 100 g | 100 g | 100 g |
| **NB** : Le 2-méthyl-5-méthoxyméthyl pyrazolo [1,5-a] pyrimidin-7-one et le 2-méthyl-6-éthoxycarbonyl pyrazolo [1,5-a] pyrimidin-7-one sont respectivement commercialisés sous les dénominations commerciales KM 00739 et KM 00318 par la société MAYBRIDG. | | | |

### Support de teinture commun n°1 :

II est identique à celui utilisé pour les exemples 1 à 3 ci-dessus.

Au moment de l'emploi, les compositions tinctoriales des exemples 5 et 6 ci-dessus ont été mélangées avec un poids égal d'une solution de peroxyde d'hydrogène à 20 volumes (6% en poids) ; la composition tinctoriale de l'exemple 7 ci-dessus a été mélangée avec un poids égal d'une solution aqueuse de persulfate d'ammonium à 6.10⁻³ mole %.

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 2 ci-dessous :

**TABLEAU 2**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **5** | 9,9 | Bois de rose très léger | Bois de rose léger |
| **6** | 9,9 | Vert bouteille | Vert bouteille |
| **7** | 9,8 | Doré blond léger | Doré blond léger |

## Revendications

1. Composition pour la teinture des fibres kératiniques et en particulier des fibres kératiniques humaines telles que les cheveux, caractérisée par le fait qu'elle comprend dans un milieu approprié pour la teinture :
- à titre de coupleur, au moins un composé pyrazolo-pyrimidineoxo de formule (I) suivante ou l'un de ses sels d'addition avec un acide : 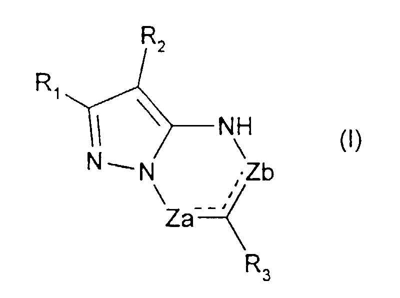 dans laquelle :
. R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle, éventuellement substitué par 1 ou 2 radicaux R tels que précédemment définis ; un hétérocycle à 5 ou 6 chaînons possédant au moins un atome d'azote, d'oxygène ou de soufre, éventuellement substitué par 1 ou 2 radicaux R tels que définis précédemment ;
lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
R₁ peut désigner aussi un atome d'halogène ; un radical acyle ; un radical sulfonyle ; un radical sulfinyle ; un radical phosphonyle, un radical carbamoyle ; un radical sulfamoyle ; un radical cyano ; un radical siloxy ; un radical amino ; un radical acylamino ; un radical acyloxy ; un radical carbamoyloxy ; un radical sulfonamide ; un radical imide ; un radical uréido ; un radical sulfamoylamino ; un radical alcoxy carbonylamino ; un radical aryloxycarbonylamino ; un radical alcoxycarbonyle ; un radical aryloxycarbonyle ; un radical carboxyle.
. R₂ représente ; un atome d'hydrogène ; un atome d'halogène tel que brome, chlore ou fluor ; un groupe acétylamido ; un radical alcoxy ; un radical aryloxy ; un radical acyloxy ; un radical arylthio ; un radical alkylthio ; un radical hétéroarylthio ; un radical hétéroaryloxy ; un radical thiocyano ; un radical N,N-diéthyl thiocarbonylthio ; un radical dodécyloxythiocarbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthylsulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazo-lydinyle ; un radical 2-oxy 1,2-dihydro 1-pyridinyle ; un alkylamido ; un arylamido ; un radical NR^{lll}R^{lV} avec R^{lll} et R^{lV} représentant, identiques ou différents, un alkyle en C₁-C₄, un hydroxyalkyle ; un carboxyle ; ou un radical alcoxycarboxylique.
R₃ a les mêmes significations indiquées que R₁.
Zₐ et Z_{b} sont différents et représentent un groupe C=O ou CR₄ où R₄ a les mêmes significations que R₁ ;
. et au moins une base d'oxydation.

2. Composition selon la revendication 1, caractérisée par le fait que les radicaux R₁ de la formule (I) sont choisis dans le groupe constitué par : un atome d'hydrogène ; un alkyle en C₁-C₄, linéaire ou ramifié ; un phényle ; un phényle substitué par un atome d'halogène, un alkyle en C₁-C₄, un alcoxy en C₁-C₄, un groupe nitro, un groupe amino, un groupe trifluorométhyle ou alkylamino en C₁-C₄ ; un radical benzyle ; un radical benzyle substitué par un atome d'halogène, un alkyle en C₁-C₄, un alcoxy en C₁-C₄, un groupe nitro, un groupe amino, un groupe trifluorométhyle ; un alkylamino en C₁-C₄ ; un hétérocycle choisi parmi le thiophène, le furane ou la pyridine ; un radical trifluorométhyle ; un radical (CH₂)ₚ-X-(CH₂)_{q}-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C₄ ; un alkylamino en C₁-C₄ ; un dialkylamino en C₁-C₄ ; un arylamino ; un radical alcoxy choisi parmi méthoxy, éthoxy et phénoxy ; un halogène choisi parmi fluor, chlore et brome ; un groupe carboxyle ; un alcoxycarbonyle en C₁-C₄ ; un phényloxycarbonyle ; méthylthio ; éthylthio ; phénylthio ; méthanesulfonyle ; cyano.

3. Composition selon la revendication 1 ou 2, caractérisée par le fait que les radicaux R₁ de la formule (I) sont choisis dans le groupe constitué par : hydrogène ; un alkyle choisi parmi méthyle, éthyle, isopropyle, ter-butyle ; un halogène choisi parmi fluor et chlore ; phényle ; toluyle ; 4-chlorophényle ; 4-méthoxyphényle ; 3-méthoxyphényle ; 2-méthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

4. Composition selon la revendication 3, caractérisée par le fait que les radicaux R₁ de la formule (I) sont choisis dans le groupe constitué par : hydrogène ; méthyle ; éthyle ; phényle ; toluyle ; 4-chlorophényle ; 4-méthoxyphényle ; benzyle ; trifluorométhyle ; chloro ; un radical méthoxy ou éthoxy ; un radical carboxyle ; un radical méthylamino ou diméthylamino ; cyano.

5. Composition selon l'une quelconque des revendications 1 à 4, caractérisée par le fait que les radicaux R₂ de la formule (I) sont choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{lll}R^{lV} avec R^{lll} et R^{lV} représentant, identiques ou différents, un alkyle en C₁-C₄ , un hydroxyalkyle en C₁-C₄ ; un carboxyle ; un radical alcoxycarboxylique en C₁-C₄.

6. Composition selon la revendication 5, caractérisée par le fait que les radicaux R₂ de la formule (I) sont choisis dans le groupe constitué par :
hydrogène ; chlore ou brome ; méthoxy ou éthoxy ; phényloxy ; 4-méthylphényloxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthyl-méthylamino ; (β-hydroxyéthyl)méthylamino.

7. Composition selon la revendication 6, caractérisée par le fait que les radicaux R₂ de la formule (I) sont choisis dans le groupe constitué par :
hydrogène ; chlore ; éthoxy ; phénoxy ; benzyloxy ; acyloxy ; acétamido ; diméthylamino.

8. Composition selon l'une quelconque des revendications 1 à 7, caractérisée par le fait que les radicaux R₃ et R₄ de la formule (I) sont choisis dans le groupe constitué par :
hydrogène ; alkyle linéaire ou ramifié en C₁-C₄, éventuellement substitué par un hydroxy ou un amino ; phényle, phényle substitué par un ou deux groupes choisis parmi halogène, alkyle en C₁-C₄, alcoxy en C₁-C₄, hydroxy, carboxyle, nitro, alkylthio en C₁-C₄, méthylènedioxy, amino, trifluorométhyle ou alkylamino en C₁-C₄ ; benzyle ; benzyle substitué par un halogène, un méthyle, un isopropyle ou méthoxy ; hydroxyalkyle en C₁-C₄ ; aminoalkyle en C₁-C₄ ; alkylaminoalkyle en C₁-C₄ ; amino ; alkylamino en C₁-C₄ ; halogène ; trifluorométhyle.

9. Composition selon la revendication 8, caractérisée par le fait que les radicaux R₃ et R₄ sont choisis dans le groupe constitué par :
hydrogène ; alkyle en C₁-C₄ ; halogène ; amino ; alkylamino en C₁-C₄ ; aryle ; benzyle ; hydroxyméthyle ou hydroxyéthyle ; aminométhyle ou aminoéthyle ; trifluorométhyle.

10. Composition selon la revendication 8 ou 9, caractérisée par le fait que les radicaux R₃ et R₄ sont choisis dans le groupe constitué par :
hydrogène, méthyle, éthyle, isopropyle, trifluorométhyle, chlore, amino, méthylamino, éthylamino, phényle, 4-chlorophényle, 4-méthoxyphényle.

11. Composition selon l'une quelconque des revendications 1 à 10, caractérisée par le fait que les composés de formule (I) sont choisis dans le groupe constitué par :
(i) les pyrazolo-[1,5-a]-pyrimidine-7-oxo de formule : 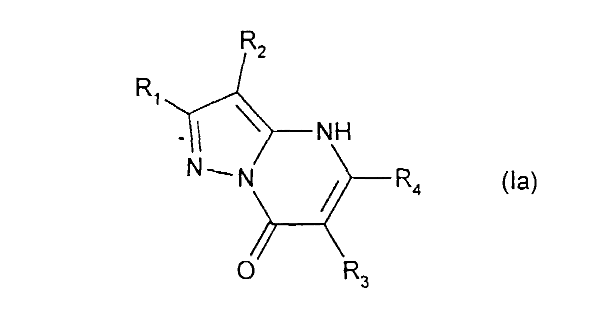
(ii) les pyrazolo-[1,5-a]-pyrimidine-5-oxo de formule : 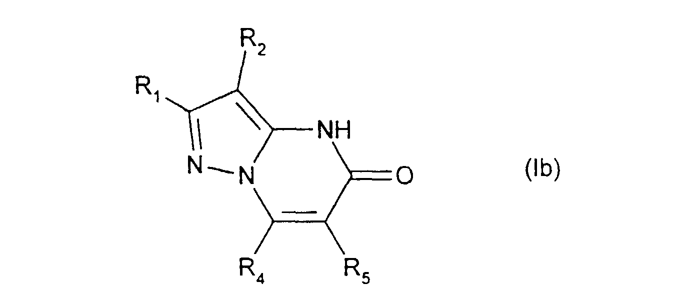 dans lesquelles les radicaux R₁, R₂, R₃ et R₄ ont les mêmes significations que celles indiquées dans l'une quelconque des revendications 1 à 10.

12. Composition selon la revendication 11, caractérisée par le fait que les composés de formule (la) sont choisis parmi ceux pour lesquels :
. R₁ désigne hydrogène, méthyle, éthyle, chlore, phényle, méthoxy, trifluorométhyle, carboxyle ou cyano ;
. R₂ désigne hydrogène, chlore ou éthoxy ;
. R₃ et R₄ désignent respectivement hydrogène et hydrogène ; hydrogène et méthyle ; méthyle et hydrogène ; hydrogène et amino ; chlore et méthyle ; chlore et amino ; carboxyle et hydrogène ; carboxyle et méthyle ; hydrogène et trifluorométhyle ou bien carboxyle et hydrogène.

13. Composition selon la revendication 11, caractérisée par le fait que les composés de formule (Ib) sont choisis parmi ceux pour lesquels les radicaux R₁, R₂, R₃ et R₄ ont les mêmes significations que celles indiquées dans la revendication 11.

14. Composition selon la revendication 11, caractérisée par le fait que les composés de formule (I) sont choisis parmi :
- le pyrazolo [1,5-a] pyrimidin-7-one,
- le 2-méthyl pyrazolo [1,5-a] pyrimidin-7-one,
- le 2-phényl pyrazolo [1,5-a] pyrimidin-7-one,
- le 2-carboxy pyrazolo [1,5-a] pyrimidin-7-one,
- le 2-éthylthio pyrazolo [1,5-a] pyrimidin-7-one,
- le 2,5-diméthyl pyrazolo [1,5-a] pyrimidin-7-one,
- le 2-phényl-5-méthyl pyrazolo [1,5-a] pyrimidin-7-one,
- le 2-carboxy-5-méthyl pyrazolo [1,5-a] pyrimidin-7-one,
- le 2-éthylthio-5-méthylpyrazolo [1,5-a] pyrimidin-7-one,
- le 2-méthyl-5-trifluorométhyl pyrazolo [1,5-a] pyrimidin-7-one,
- le 2-phényl-5-trifluorométhyl pyrazolo [1,5-a] pyrimidin-7-one,
- le 2-carboxy-5-trifluorométhyl pyrazolo [1,5-a] pyrimidin-7-one,
- le 2-éthylthio-5-trifluorométhyl pyrazolo [1,5-a] pyrimidin-7-one,
- le 5-trifluorométhyl pyrazolo [1,5-a] pyrimidin-7-one,
- le 5-méthyl pyrazolo [1,5-a] pyrimidin-7-one,
- le 6-carboxy pyrazolo [1,5-a] pyrimidin-7-one,
- le 6-carboxy-2-méthyl pyrazolo [1,5-a] pyrimidin-7-one,
- le 6-carboxy-2-phényl pyrazolo [1,5-a] pyrimidin-7-one,
- le 6-carboxy-2-éthylthio pyrazolo [1,5-a] pyrimidin-7-one,
- le 2,6-dicarboxy pyrazolo [1,5-a] pyrimidin-7-one,
- le 2-méthyl-6-éthoxycarbonyl pyrazolo [1,5-a] pyrimidin-7-one,
- le 2-(2'-furyl)-6-méthyl pyrazolo [1,5-a] pyrimidin-7-one,
- le 2-(2'-thienyl)-6méthyl pyrazolo [1,5-a] pyrimidin-7-one,
- le 3-éthoxycarbonyl-6-méthyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
- le pyrazolo [1,5-a] pyrimidin-5-one,
- le 2-méthyl pyrazolo [1,5-a] pyrimidin-5-one,
- 2,7-diméthyl pyrazolo [1,5-a] pyrimidin-5-one,
- le 2-phényl pyrazolo [1,5-a] pyrimidin-5-one,
- le 2-éthylthio pyrazolo [1,5-a] pyrimidin-5-one,
- le 2-carboxy pyrazolo [1,5-a] pyrimidin-5-one,
- le 7-amino pyrazolo [1,5-a] pyrimidin-5-one,
- le 7-amino-2-méthyl pyrazolo [1,5-a] pyrimidin-5-one,
- le 7-amino-2-phényl pyrazolo [1,5-a] pyrimidin-5-one,
- le 7-amino--2-éthylthio pyrazolo [1,5-a] pyrimidin-5-one,
- le 7-amino-2-carboxy pyrazolo [1,5-a] pyrimidin-5-one,
et leurs sels d'addition avec un acide.

15. Composition selon l'une quelconque des revendications précédentes, caractérisée par le fait que les sels d'addition avec un acide des composés de formule (I) sont choisis parmi les chlorhydrates, les bromhydrates, les sulfates, les tartrates, les benzènesulfonates, les lactates, les tosylates et les acétates.

16. Composition selon l'une quelconque des revendications précédentes, caractérisée par le fait que le ou les composés de formule (I) représentent de 0,0005 à 12 % en poids du poids total de la composition tinctoriale.

17. Composition selon la revendication 16, caractérisée par le fait que le ou les composés de formule (I) représentent de 0,0005 à 12 % en poids du poids total de la composition tinctoriale.

18. Composition selon l'une quelconque des revendications précédentes, caractérisée par le fait que la ou les bases d'oxydation sont choisies parmi les paraphénylènediamines, les bis-paraphénylènediamines, les paraaminophénols, les ortho-aminophénols, les bases hétérocycliques, et leurs sels d'addition avec un acide.

19. Composition selon l'une quelconque des revendications précédentes, caractérisée par le fait que la ou les bases d'oxydation représentent de 0,0005 à 12 % en poids environ du poids total de la composition tinctoriale.

20. Composition selon la revendication 19, caractérisée par le fait que la ou les bases d'oxydation représentent de 0,0005 à 6 % en poids environ du poids total de la composition tinctoriale.

21. Composition selon l'une quelconque des revendications précédentes, caractérisée par le fait qu'elle renferme en outre un ou plusieurs coupleurs additionnels différents des composés de formule (I) et/ou un ou plusieurs colorants directs.

22. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcools inférieurs en C₁-C₄, le glycérol, les glycols et éthers de glycols, les alcools aromatiques, les produits analogues et leurs mélanges.

23. Composition selon l'une quelconque des revendications précédentes, caractérisée par le fait qu'elle présente un pH compris entre 3 et 12.

24. Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

25. Utilisation des composés de formule (I) ou de leurs sels d'addition avec un acide tel que définis à l'une quelconque des revendications 1 à 15, à titre de coupleurs dans des compositions pour la teinture d'oxydation des fibres kératiniques et en particulier des fibres kératiniques humaines telles que les cheveux, en association avec au moins une base d'oxydation.

26. Procédé de teinture d'oxydation des fibres kératiniques et en particulier des fibres kératiniques humaines telles que les cheveux, caractérisé par le fait qu'on applique sur ces fibres au moins une composition tinctoriale telle que définie à l'une quelconque des revendications 1 à 24, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

27. Procédé selon la revendication 26, caractérisé par le fait que l'agent oxydant est choisi parmi le peroxyde d'hydrogène, le peroxyde d'urée, les bromates de métaux alcalins, les persels tels que les perborates et persulfates.

28. Dispositifs à plusieurs compartiments, ou "kit" de teinture à plusieurs compartiments, dont un premier compartiment renferme une composition tinctoriale telle que définie à l'une quelconque des revendications 1 à 24 et un second compartiment renferme une composition oxydante.

## Patentansprüche

1. Zusammensetzung zum Färben von Keratinfasern und insbesondere menschlichen Keratinfasern, wie dem Haar, dadurch gekennzeichnet, daß sie in einem zum Färben geeigneten Medium enthält:
- als Kuppler mindestens eine Pyrazolo-pyrimidinoxo-Verbindung der folgenden Formel (I) oder eines der Additionssalze dieser Verbindungen mit einer Säure: 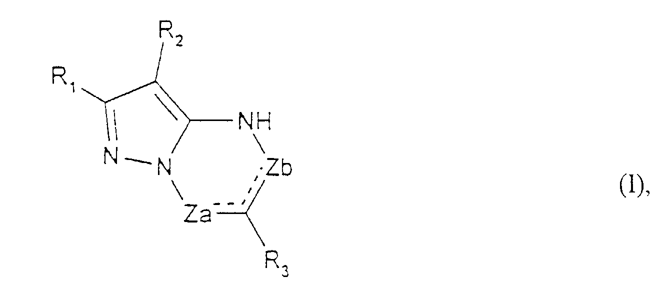 worin:
R₁ bedeutet: Wasserstoff; eine geradkettige oder verzweigte C₁₋₂₀-Alkylgruppe, die gegebenenfalls mit einer oder zwei Gruppen R substituiert ist, die unter Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, Alkoxycarbonyl oder Acyl ausgewählt ist; eine Arylgruppe, die gegebenenfalls mit einer oder zwei oben definierten Gruppen R substituiert ist; einen 5- oder 6-gliedrigen Heterocyclus, der mindestens ein Stickstoff-, Sauerstoff- oder Schwefelatom aufweist und gegebenenfalls mit einer oder zwei oben definierten Gruppen R substituiert ist;
wenn die Gruppe R₁ eine Alkylgruppe, eine Arylgruppe oder einen 5- oder 6-gliedrigen Heterocyclus bedeutet (wie oben definiert), kann sie über ein Sauerstoff-, Stickstoff- oder Schwefelatom an das Kohlenstoffatom des Ringes gebunden sein ( in diesem Fall wird R₁ zu XR₁ mit X = O, NH oder S);
R₁ kann auch bedeuten: Halogen; Acyl; Sulfonyl; Sulfinyl; Phosphonyl; Carbamoyl; Sulfamoyl; Cyano; Siloxy; Amino; Acylamino; Acyloxy; Carbamoyloxy; Sulfonamid; Imid; Ureido; Sulfamoylamino; Alkoxycarbonylamino; Aryloxycarbonylamino; Alkoxycarbonyl; Aryloxycarbonyl; Carboxy;
R₂ bedeutet: Wasserstoff; Halogen, wie Brom, Chlor oder Fluor; Acetylamido; Alkoxy; Aryloxy; Acyloxy; Arylthio; Alkylthio; Heteroarylthio; Heteroaryloxy; Thiocyano; N,N-Diethyl-thiocarbonylthio; Dodecyloxythiocarbonylthio; Benzolsulfonamido; N-Ethyltoluolsulfonamido; Pentafluorbutanamido; 2,3,4,5,6-Pentafluorbenzamido; p-Cyanophenylureido; N,N-Diethylsulfamoylamino; Pyrazolyl; Imidazolyl; Triazolyl; Tetrazolyl; Benzimidazolyl; 1-Benzyl-5-ethoxy-3-hydantoinyl; l-Benzyl-3-hydantoinyl; 5,5-Dimethyl-2,4-dioxo-3-oxazolidinyl; 2-Oxy-1,2-dihydro-1-pyridinyl; Alkylamido; Arylamido; NR^{lll}R^{lV} und R^{lV}, die identisch oder voneinander verschieden sind, C₁₋₄-Alkyl oder Hydroxyalkyl bedeuten; Carboxy; Alkoxycarboxy;
R₃ bedeutet: die für R₁ angegebenen Gruppen;
Zₐ und Z_{b} voneinander verschieden sind und eine Gruppe C=O oder CR₄ bedeuten, wobei R₄ die oben für die Gruppe R₁ angegebenen Bedeutungen aufweist;
und
- mindestens eine Oxidationsbase.

2. Zusammensetzung nach Anspruch 1, dadurch gekennzeichnet, daß die Gruppen R₁ der Formel (I) ausgewählt sind unter:
Wasserstoff; einer geradkettigen oder verzweigten C₁₋₄-Alkylgruppe; Phenyl; einer Phenylgruppe, die mit einem Halogenatom, C₁₋₄-Alkyl, C₁₋₄-Alkoxy, Nitro, Amino, Trifluormethyl oder C₁₋₄-Alkylamino substituiert ist; Benzyl; einer Benzylgruppe, die mit einem Halogenatom, C₁₋₄-Alkyl, C₁₋₄-Alkoxy, Nitro, Amino oder Trifluormethyl substituiert ist; C₁₋₄-Alkylamino; einem Heterocyclus, der unter Thiophen, Furan oder Pyridin ausgewählt ist; Trifluormethyl; einer Gruppe (CH₂)ₚ-X-(CH₂)_{q}-OR', worin p und q identisch oder voneinander verschieden sind und ganze Zahlen im Bereich von 1 bis 3 bedeuten und R' H oder Methyl und X Sauerstoff oder NR" mit R" = Wasserstoff oder Methyl bedeutet; C₁₋₄-Hydroxyalkyl; C₁₋₄-Aminoalkyl; C₁₋₄-Alkylamino; C₁₋₄-Dialkylamino; Arylamino; einer Alkoxygruppe, die unter Methoxy, Ethoxy und Phenoxy ausgewählt ist; einem Halogenatom, das unter Fluor, Chlor und Brom ausgewählt ist; Carboxy; C₁₋₄-Alkoxycarbonyl; Phenyloxycarbonyl; Methylthio; Ethylthio; Phenylthio; Methansulfonyl; Cyano.

3. Zusammensetzung nach Anspruch 1 oder 2, dadurch gekennzeichnet, daß die Gruppen R₁ der Formel (I) ausgewählt sind unter:
Wasserstoff; einer Alkylgruppe, die unter Methyl, Ethyl, Isopropyl und tert.-Butyl ausgewählt ist; einem Halogenatom, das unter Fluor und Chlor ausgewählt ist; Phenyl; Toluyl; 4-Chlorphenyl; 4-Methoxyphenyl; 3-Methoxyphenyl; 2-Methoxyphenyl; Benzyl; einem Heterocyclus, der unter Pyridyl, Furyl oder Thienyl ausgewählt ist; Trifluormethyl; Hydroxymethyl; Aminomethyl; Methoxy oder Ethoxy; Methylamino oder Ethylamino oder Dimethylamino; Carboxy; Methoxycarbonyl oder Ethoxycarbonyl; Cyano.

4. Zusammensetzung nach Anspruch 3, dadurch gekennzeichnet, daß die Gruppen R₁ der Formel (I) ausgewählt sind unter:
Wasserstoff; Methyl; Ethyl; Phenyl; Toluyl; 4-Chlorphenyl; 4-Methoxyphenyl; Benzyl; Trifluormethyl; Chlor; Methoxy oder Ethoxy; Carboxy; Methylamino oder Dimethylamino; Cyano.

5. Zusammensetzung nach einem der Ansprüche 1 bis 4, dadurch gekennzeichnet, daß die Gruppen R₂ der Formel (I) ausgewählt sind unter:
Wasserstoff; C₁₋₄-Alkoxy; Phenoxy; einer Phenoxygruppe, die mit Halogen, C₁₋₄-Alkyl, Carboxy oder Trifluormethyl substituiert ist; Acyloxy; Benzyloxy; C₁₋₄-Alkylthio; Phenylthio; einer Phenylthiogruppe, die mit Halogen, C₁₋₄-Alkyl, Carboxy oder Trifluormethyl substituiert ist; C₁₋₄-Alkylamido; Phenylamido; NR^{lll}R^{lV}, worin R^{lll} und R^{lV}, die identisch oder voneinander verschieden sind, C₁₋₄-Alkyl oder C₁₋₄-Hydroxyalkyl bedeuten; Carboxy; C₁₋₄-Alkoxycarboxy.

6. Zusammensetzung nach Anspruch 5, dadurch gekennzeichnet, daß die Gruppen R₂ der Formel (I) ausgewählt sind unter:
Wasserstoff; Chlor oder Brom; Methoxy oder Ethoxy; Phenoxy; 4-Methylphenoxy; Acyloxy; Benzyloxy; Methylthio oder Ethylthio; Phenylthio; 4-Methylphenylthio; 2-tert.-Butylphenylthio; Acetamido; Phenylacetamido; Dimethylamino; Diethylamino; Ethylmethylamino; (β-Hydroxyethyl)methylamino.

7. Zusammensetzung nach Anspruch 6, dadurch gekennzeichnet, daß die Gruppen R₂ der Formel (I) ausgewählt sind unter:
Wasserstoff; Chlor; Ethoxy; Phenoxy; Benzyloxy; Acyloxy; Acetamido; und Dimethylamino.

8. Zusammensetzung nach einem der Ansprüche 1 bis 7, dadurch gekennzeichnet, daß die Gruppen R₃ und R₄ der Formel (I) ausgewählt sind unter:
Wasserstoff; einer geradkettigen oder verzweigten C₁₋₄-Alkylgruppe, die gegebenenfalls mit Hydroxy oder Amino substituiert ist; Phenyl; einer Phenylgruppe, die mit l oder 2 Gruppen substituiert ist, die unter Halogen, C₁₋₄-Alkyl, C₁₋₄-Alkoxy, Hydroxy, Carboxy, Nitro, C₁₋₄-Alkylthio, Methylendioxy, Amino, Trifluormethyl oder C₁₋₄-Alkylamino ausgewählt sind; Benzyl; einer Benzylgruppe, die mit Halogen, Methyl, Isopropyl oder Methoxy substituiert ist; C₁₋₄-Hydroxyalkyl; C₁₋₄-Aminoalkyl; C₁₋₄-Alkylaminoalkyl; Amino; C₁₋₄-Alkylamino; Halogen; Trifluormethyl.

9. Zusammensetzung nach Anspruch 8, dadurch gekennzeichnet, daß die Gruppen R₃ und R₄ ausgewählt sind unter:
Wasserstoff; C₁₋₄-Alkyl; Halogen; Amino; C₁₋₄-Alkylamino; Aryl; Benzyl; Hydroxymethyl oder Hydroxyethyl; Aminomethyl oder Aminoethyl; Trifluormethyl.

10. Zusammensetzung nach Anspruch 8 oder 9, dadurch gekennzeichnet, daß die Gruppen R₃ und R₄ ausgewählt sind unter:
Wasserstoff, Methyl, Ethyl, Isopropyl, Trifluormethyl, Chlor, Amino, Methylamino, Ethylamino, Phenyl, 4-Chlorphenyl und 4-Methoxyphenyl.

11. Zusammensetzung nach einem der Ansprüche 1 bis 10, dadurch gekennzeichnet, daß die erfindungsgemäßen Verbindungen der Formel (I) ausgewählt sind unter:
(i) den Pyrazolo[1,5-a]pyrimidin-7-oxo-Verbindungen der Formel: 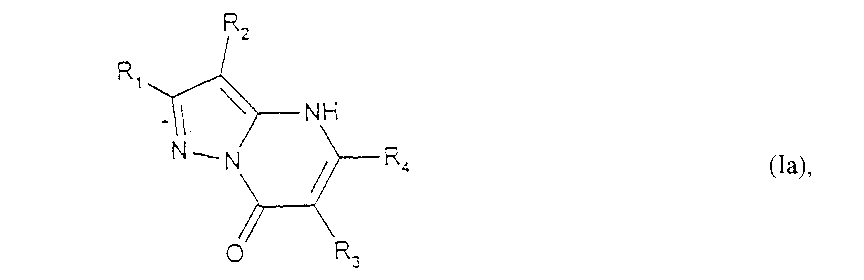
(ii) den Pyrazolo[1,5-a]pyrimidin-5-oxo-Verbindungen der Formel: 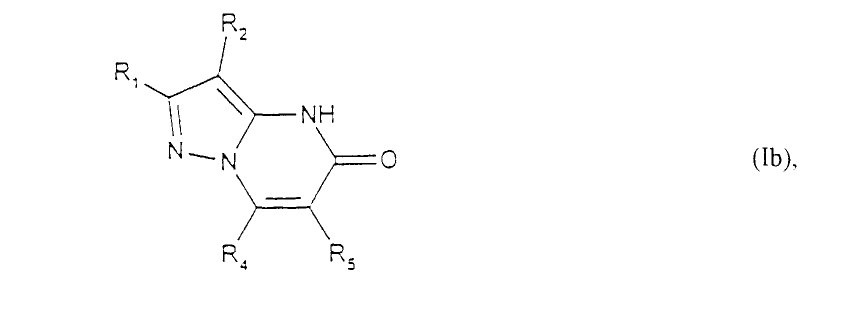 worin die Gruppen R₁, R₂, R₃ und R₄ die oben in einem der Ansprüche 1 bis 10 angegebenen Bedeutungen aufweisen.

12. Zusammensetzung nach Anspruch 11, dadurch gekennzeichnet, daß die Verbindungen der Formel (Ia) unter den Verbindungen ausgewählt sind, worin bedeuten:
R₁ Wasserstoff, Methyl, Ethyl, Chlor, Phenyl, Methoxy, Trifluormethyl, Carboxy oder Cyano;
R₂ Wasserstoff, Chlor oder Ethoxy;
R₃ bzw. R₄ Wasserstoffbzw. Wasserstoff; Wasserstoffbzw. Methyl; Methyl bzw. Wasserstoff; Wasserstoffbzw. Amino; Chlor bzw. Methyl; Chlor bzw. Amino; Carboxy bzw. Wasserstoff; Carboxy bzw. Methyl; Wasserstoffbzw. Trifluormethyl oder Carboxy bzw. Wasserstoff.

13. Zusammensetzung nach Anspruch 11, dadurch gekennzeichnet, daß die Verbindungen der Formel (Ib) unter den Verbindungen ausgewählt sind, worin die Gruppen R₁, R₂, R₃ und R₄ die in Anspruch 11 angegebenen Bedeutungen aufweisen.

14. Zusammensetzung nach Anspruch 11, dadurch gekennzeichnet, daß die Verbindungen der Formel (I) ausgewählt sind unter:
- Pyrazolo[1,5-a]pyrimidin-7-on,
- 2-Methyl-pyrazolo[1,5-a]pyrimidin-7-on,
- 2-Phenyl-pyrazolo[1,5-a]pyrimidin-7-on,
- 2-Carboxy-pyrazolo[1,5-a]pyrimidin-7-on,
- 2-Ethylthio-pyrazolo[1,5-a]pyrimidin-7-on,
- 2,5-Dimethyl-pyrazolo[1,5-a]pyrimidin-7-on,
- 2-Phenyl-5-methyl-pyrazolo[1,5-a]pyrimidin-7-on,
- 2-Carboxy-5-methyl-pyrazolo[1,5-a]pyrimidin-7-on,
- 2-Ethylthio-5-methyl-pyrazolo[1,5-a]pyrimidin-7-on,
- 2-Methyl-5-trifluormethyl-pyrazolo[1,5-a]pyrimidin-7-on,
- 2-Phenyl-5-trifluormethyl-pyrazolo[1,5-a]pyrimidin-7-on,
- 2-Carboxy-5-trifluormethyl-pyrazolo[1,5-a]pyrimidin-7-on,
- 2-Ethylthio-5-trifluormethyl-pyrazolo[1,5-a]pyrimidin-7-on,
- 5-Trifluormethyl-pyrazolo[1,5-a]pyrimidin-7-on,
- 5-Methyl-pyrazolo[1,5-a]pyrimidin-7-on,
- 6-Carboxy-pyrazolo[1,5-a]pyrimidin-7-on,
- 6-Carboxy-2-methyl-pyrazolo[1,5-a]pyrimidin-7-on,
- 6-Carboxy-2-phenyl-pyrazolo[1,5-a]pyrimidin-7-on,
- 6-Carboxy-2-ethylthio-pyrazolo[1,5-a]pyrimidin-7-on,
- 2,6-Dicarboxy-pyrazolo[1,5-a]pyrimidin-7-on,
- 2-Methyl-6-ethoxycarbonyl-pyrazolo[1,5-a]pyrimidin-7-on,
- 2-(2'-Furyl)-6-methyl-pyrazolo[ 1,5-a]pyrimidin-7-on,
- 2-(2'-Thienyl)-6-methyl-pyrazolo[1,5-a]pyrimidin-7-on,
- 3-Ethoxycarbonyl-6-methyl-pyrazolo[1,5-a]pyrimidin-7-on,
- 2-Methyl-5-methoxymethyl-pyrazolo[1,5-a]pyrimidin-7-on,
- 2-tert.-Butyl-5-trifluormethyl-pyrazolo[1,5-a]pyrimidin-7-on,
- Pyrazolo[1,5-a]pyrimidin-5-on,
- 2-Methyl-pyrazolo[1,5-a]pyrimidin-5-on,
- 2,7-Dimethyl-pyrazolo[1,5-a]pyrimidin-5-on,
- 2-Phenyl-pyrazolo[1,5-a]pyrimidin-5-on,
- 2-Ethylthio-pyrazolo[1,5-a]pyrimidin-5-on,
- 2-Carboxy-pyrazolo[1,5-a]pyrimidin-5-on,
- 7-Amino-pyrazolo[ 1,5-a]pyrimidin-5-on,
- 7-Amino-2-methyl-pyrazolo[1,5-a]pyrimidin-5-on,
- 7-Amino-2-phenyl-pyrazolo[1,5-a]pyrimidin-5-on,
- 7-Amino-2-ethylthio-pyrazolo[ 1,5-a]pyrimidin-5-on,
- 7-Amino-2-carboxy-pyrazolo[1,5-a]pyrimidin-5-on, und
deren Additionssalze mit einer Säure.

15. Zusammensetzung nach einem der vorhergehenden Ansprüche, dadurch gekennzeichnet, daß die Additionssalze der Verbindungen der Formel (I) mit einer Säure unter den Hydrochloriden, Hydrobromiden, Sulfaten, Tartraten, Benzolsulfonaten, Lactaten, Tosylaten und Acetaten ausgewählt sind.

16. Zusammensetzung nach einem der vorhergehenden Ansprüche, dadurch gekennzeichnet, daß die Verbindung(en) der Formel (I) 0,0005 bis 12 Gew.-% des Gesamtgewichts der Färbemittelzusammensetzung ausmachen.

17. Zusammensetzung nach Anspruch 16, dadurch gekennzeichnet, daß die Verbindung(en) der Formel (I) 0,0005 bis 12 Gew.-% des Gesamtgewichts der Färbemittelzusammensetzung ausmachen.

18. Zusammensetzung nach einem der vorhergehenden Ansprüche, dadurch gekennzeichnet, daß die Oxidationsbase(n) unter den p-Phenylendiaminen, Bis-paraphenylendiaminen, p-Aminophenolen, o-Aminophenolen, heterocyclischen Basen und deren Additionssalzen mit einer Säure ausgewählt sind.

19. Zusammensetzung nach einem der vorhergehenden Ansprüche, dadurch gekennzeichnet, daß die Oxidationsbase(n) etwa 0,0005 bis 12 Gew.-% des Gesamtgewichts der Färbemittelzusammensetzung ausmachen.

20. Zusammensetzung nach Anspruch 19, dadurch gekennzeichnet, daß die Oxidationsbase(n) etwa 0,0005 bis 6 Gew.-% des Gesamtgewichts der Färbemittelzusammensetzung ausmachen.

21. Zusammensetzung nach einem der vorhergehenden Ansprüche, dadurch gekennzeichnet, daß sie ferner einen oder mehrere zusätzliche Kuppler, die von den Verbindungen der Formel (I) verschieden sind, und/oder einen oder mehrere Direktfarbstoffe enthält.

22. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

23. Zusammensetzung nach einem der vorhergehenden Ansprüche, dadurch gekennzeichnet, daß sie einen pH-Wert im Bereich von 3 bis 12 aufweist.

24. Zusammensetzung nach einem der vorhergehenden Ansprüche, dadurch gekennzeichnet, daß sie als Flüssigkeit, Creme oder Gel oder in beliebigen weiteren Formen, die zur Durchführung einer Färbung von Keratinfasern und insbesondere von menschlichem Haar geeignet sind, vorliegt.

25. Verwendung von Verbindungen der Formel (I) oder von Additionssalzen dieser Verbindungen mit einer Säure nach einem der Ansprüche 1 bis 15 als Kuppler in Zusammensetzungen zum oxidativen Färben von Keratinfasern und insbesondere menschlichen Keratinfasern, wie dem Haar, in Kombination mit mindestens einer Oxidationsbase.

26. Verfahren zum oxidativen Färben von Keratinfasern und insbesondere menschlichen Keratinfasern, wie dem Haar, dadurch gekennzeichnet, daß auf die Fasern mindestens eine Färbemittelzusammensetzung nach einem der Ansprüche 1 bis 24 aufgetragen wird und die Farbe bei einem sauren, neutralen oder alkalischen pH-Wert mit einem Oxidationsmittel entwickelt wird, das bei der Anwendung zu der Färbemittelzusammensetzung gegeben wird oder das in einer oxidierenden Zusammensetzung vorliegt, die gleichzeitig oder getrennt davon anschließend aufgetragen wird.

27. Verfahren nach Anspruch 26, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

28. Vorrichtung mit mehreren Abteilungen oder "Kit" zum Färben mit mehreren Abteilungen, wobei eine erste Abteilung eine Färbemittelzusammensetzung nach einem der Ansprüche 1 bis 24 und eine zweite Abteilung eine oxidierende Zusammensetzung enthält.

## Claims

1. Composition for dyeing keratin fibres, and in particular human keratin fibres such as the hair, characterized in that it comprises, in a medium which is suitable for dyeing:
- as coupler, at least one pyrazolopyrimidinoxo compound of formula (I) below, or one of the addition salts thereof with an acid: 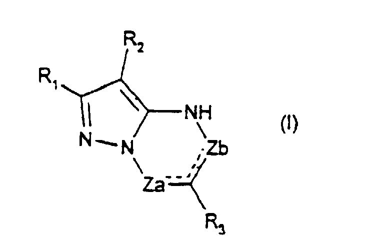 in which:
R₁ represents: a hydrogen atom; a linear or branched C₁-C₂₀ alkyl radical optionally substituted with 1 or 2 radicals R chosen from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, alkoxycarbonyl, acyl; an aryl radical optionally substituted with 1 or 2 radicals R as defined above; a 5- or 6-membered heterocycle having at least one nitrogen, oxygen or sulphur atom optionally substituted with 1 or 2 radicals R as defined above;
when R₁ denotes an alkyl radical, an aryl radical or a 5- or 6-membered heterocycle (defined above), it can be linked to the carbon atom of the ring via an oxygen, nitrogen or sulphur atom (in this case, R₁ becomes XR₁ with X = O, NH, S) ;
R₁ can also denote a halogen atom; an acyl radical; a sulphonyl radical; a sulphinyl radical; a phosphonyl radical; a carbamoyl radical; a sulphamoyl radical; a cyano radical; a siloxy radical; an amino radical; an acylamino radical; an acyloxy radical; a carbamoyloxy radical; a sulphonamide radical; an imide radical; a ureido radical; a sulphamoylamino radical; an alkoxycarbonylamino radical; an aryloxycarbonylamino radical; an alkoxycarbonyl radical; an aryloxycarbonyl radical; a carboxyl radical;
R₂ represents: a hydrogen atom; a halogen atom such as bromine, chlorine or fluorine; an acetylamido group; an alkoxy radical; an aryloxy radical; an acyloxy radical; an arylthio radical; an alkylthio radical; a heteroarylthio radical; a heteroaryloxy radical; a thiocyano radical; an N,N-diethylthiocarbonylthio radical; a dodecyloxythiocarbonylthio radical; a benzenesulphonamido radical; an N-ethyltoluenesulphonamido radical; a pentafluorobutanamido radical; a 2,3,4,5,6-pentafluorobenzamido radical; a p-cyanophenylureido radical; an N,N-diethylsulphamoylamino radical; a pyrazolyl radical; an imidazolyl radical; a triazolyl radical; a tetrazolyl radical, a benzimidazolyl radical; a 1-benzyl-5-ethoxy-3-hydantoinyl radical; a 1-benzyl-3-hydantoinyl radical; 5,5-dimethyl-2,4-dioxo-3-oxazolidinyl; a 2-oxy-1,2-dihydro-1-pyridyl radical; an alkylamido; an arylamido; a radical NR^{III}R^{IV} with R^{III} and R^{IV}, which may be identical or different, representing a C₁-C₄ alkyl, a hydroxyalkyl; a carboxyl; or an alkoxycarboxylic radical;
R₃ has the same meanings mentioned as R₁;
Zₐ and Z_{b} are different and represent a C=O group or CR₄ where R₄ has the same meanings as R₁;
and at least one oxidation base.

2. Composition according to Claim 1, characterized in that the radicals R₁ of formula (I) are chosen from the group consisting of:
a hydrogen atom; a linear or branched C₁-C₄ alkyl; a phenyl; a phenyl substituted with a halogen atom; a C₁-C₄ alkyl, a C₁-C₄ alkoxy, a nitro group, an amino group, a trifluoromethyl group or a C₁-C₄ alkylamino group; a benzyl radical; a benzyl radical substituted with a halogen atom, a C₁-C₄ alkyl, a C₁-C₄ alkoxy, a nitro group, an amino group, a trifluoromethyl group; a C₁-C₄ alkylamino; a heterocycle chosen from thiophene, furan and pyridine; a trifluoromethyl radical; a radical (CH₂)ₚ-X-(CH₂)_{q}-OR' where p and q are integers, which may be identical or different, between 1 and 3, R' represents H or methyl and X denotes an oxygen atom or a group NR" with R" denoting hydrogen or methyl; a C₁-C₄ hydroxyalkyl; a C₁-C₄ aminoalkyl; a C₁-C₄ alkylamino; a C₁-C₄ dialkylamino; an arylamino; an alkoxy radical chosen from methoxy, ethoxy and phenoxy; a halogen chosen from fluorine, chlorine and bromine; a carboxyl group; a (C₁-C₄)alkoxycarbonyl; a phenyloxycarbonyl; methylthio; ethylthio; phenylthio; methanesulphonyl; cyano.

3. Composition according to Claim 1 or 2, characterized in that the radicals R₁ of formula (I) are chosen from the group consisting of:
hydrogen; an alkyl chosen from methyl, ethyl, isopropyl, tert-butyl; a halogen chosen from fluorine and chlorine; phenyl; toluyl; 4-chlorophenyl; 4-methoxyphenyl; 3-methoxyphenyl; 2-methoxyphenyl; benzyl; a heterocycle chosen from pyridyl, furyl and thienyl; trifluoromethyl; hydroxymethyl; aminomethyl; methoxy or ethoxy; methylamino or ethylamino or dimethylamino; carboxyl; methoxycarbonyl or ethoxycarbonyl; cyano.

4. Composition according to Claim 3, characterized in that the radicals R₁ of formula (I) are chosen from the group consisting of:
hydrogen; methyl; ethyl; phenyl; toluyl; 4-chlorophenyl; 4-methoxyphenyl; benzyl; trifluoromethyl; chloro; a methoxy or ethoxy radical; a carboxyl radical; a methylamino or dimethylamino radical; cyano.

5. Composition according to any one of Claims 1 to 4, characterized in that the radicals R₂ of formula (I) are chosen from the group consisting of:
a hydrogen atom; a C₁-C₄ alkoxy; phenoxy; phenoxy substituted with a halogen atom, a C₁-C₄ alkyl, a carboxyl, a trifluoromethyl group; an acyloxy radical; benzyloxy; C₁-C₄ alkylthio; phenylthio; phenylthio substituted with a halogen atom, a C₁-C₄ alkyl, a carboxyl, a trifluoromethyl group; a C₁-C₄ alkylamido; phenylamido; a radical NR^{III}R^{IV} with R^{III} and R^{IV}, which may be identical or different, representing a C₁-C₄ alkyl, a C₁-C₄ hydroxyalkyl; a carboxyl; a C₁-C₄ alkoxycarboxylic radical.

6. Composition according to Claim 5, characterized in that the radicals R₂ of formula (I) are chosen from the group consisting of:
hydrogen; chlorine or bromine; methoxy or ethoxy; phenyloxy; 4-methylphenyloxy; acyloxy; benzyloxy; methylthio or ethylthio; phenylthio; 4-methylphenylthio; 2-tert-butylphenylthio; acetamido; phenylacetamido; dimethylamino; diethylamino; ethylmethylamino; (β-hydroxyethyl)methylamino.

7. Composition according to Claim 6, characterized in that the radicals R₂ of formula (I) are chosen from the group consisting of:
hydrogen; chlorine; ethoxy; phenoxy; benzyloxy; acyloxy; acetamido; dimethylamino.

8. Composition according to any one of Claims 1 to 7, characterized in that the radicals R₃ and R₄ of formula (I) are chosen from the group consisting of:
hydrogen; linear or branched C₁-C₄ alkyl optionally substituted with a hydroxyl or an amino; phenyl, phenyl substituted with one or two groups chosen from halogen, C₁-C₄ alkyl, C₁-C₄ alkoxy, hydroxyl, carboxyl, nitro, C₁-C₄ alkylthio, methylenedioxy, amino, trifluoromethyl or C₁-C₄ alkylamino; benzyl; benzyl substituted with a halogen, a methyl, an isopropyl or methoxy; C₁-C₄ hydroxyalkyl; C₁-C₄ aminoalkyl; C₁-C₄ alkylaminoalkyl; amino; C₁-C₄ alkylamino; halogen; trifluoromethyl.

9. Composition according to Claim 8, characterized in that the radicals R₃ and R₄ are chosen from the group consisting of:
hydrogen; C₁-C₄ alkyl; halogen; amino; C₁-C₄ alkylamino; aryl; benzyl; hydroxymethyl or hydroxyethyl; aminomethyl or aminoethyl; trifluoromethyl.

10. Composition according to Claim 8 or 9, characterized in that the radicals R₃ and R₄ are chosen from the group consisting of:
hydrogen, methyl, ethyl, isopropyl, trifluoromethyl, chlorine, amino, methylamino, ethylamino, phenyl, 4-chlorophenyl, 4-methoxyphenyl.

11. Composition according to any one of Claims 1 to 10, characterized in that the compounds of formula (I) are chosen from the group consisting of:
(i) pyrazolo[1,5-a]pyrimidin-7-oxo compounds of formula: 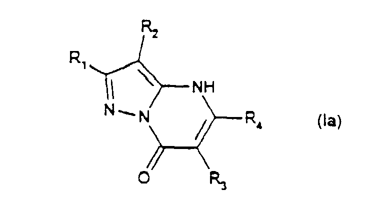
(ii) pyrazolo[1,5-a]pyrimidin-5-oxo compounds of formula: 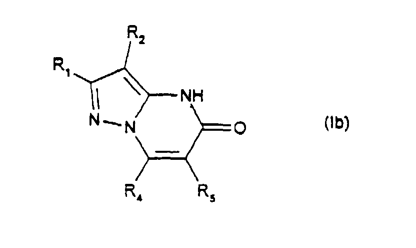 in which the radicals R₁, R₂, R₃ and R₄ have the same meanings as those mentioned in any one of Claims 1 to 10.

12. Composition according to Claim 11, characterized in that the compounds of formula (Ia) are chosen from those for which:
R₁ denotes hydrogen, methyl, ethyl, chlorine, phenyl, methoxy, trifluoromethyl, carboxyl or cyano;
R₂ denotes hydrogen, chlorine or ethoxy;
R₃ and R₄ respectively denote: hydrogen and hydrogen; hydrogen and methyl; methyl and hydrogen; hydrogen and amino; chlorine and methyl; chlorine and amino; carboxyl and methyl; hydrogen and trifluoromethyl, or carboxyl and hydrogen.

13. Composition according to Claim 11, characterized in that the compounds of formula (Ib) are chosen from those for which the radicals R₁, R₂, R₃ and R₄ have the same meanings as those mentioned in Claim 11.

14. Composition according to Claim 11, characterized in that the compounds of formula (I) are chosen from:
- pyrazolo[1,5-a]pyrimidin-7-one,
- 2-methylpyrazolo[1,5-a]pyrimidin-7-one,
- 2-phenylpyrazolo[1,5-a]pyrimidin-7-one,
- 2-carboxypyrazolo[1,5-a]pyrimidin-7-one,
- 2-ethylthiopyrazolo[1,5-a]pyrimidin-7-one,
- 2,5-dimethylpyrazolo[1,5-a]pyrimidin-7-one,
- 2-phenyl-5-methylpyrazolo[1,5-a]pyrimidin-7-one,
- 2-carboxy-5-methylpyrazolo[1,5-a]pyrimidin-7-one,
- 2-ethylthio-5-methylpyrazolo[1,5-a]pyrimidin-7-one,
- 2-methyl-5-trifluoromethylpyrazolo[1,5-a]pyrimidin-7-one,
- 2-phenyl-5-trifluoromethylpyrazolo[1,5-a]pyrimidin-7-one,
- 2-carboxy-5-trifluoromethylpyrazolo[1,5-a]pyrimidin-7-one,
- 2-ethylthio-5-trifluoromethylpyrazolo[1,5-a]pyrimidin-7-one,
- 5-trifluoromethylpyrazolo[1,5-a]pyrimidin-7-one,
- 5-methylpyrazolo[1,5-a]pyrimidin-7-one,
- 6-carboxypyrazolo[1,5-a]pyrimidin-7-one,
- 6-carboxy-2-methylpyrazolo[1,5-a]pyrimidin-7-one,
- 6-carboxy-2-phenylpyrazolo[1,5-a]pyrimidin-7-one,
- 6-carboxy-2-ethylthiopyrazolo[1,5-a]pyrimidin-7-one,
- 2,6-dicarboxypyrazolo[1,5-a]pyrimidin-7-one,
- 2-methyl-6-ethoxycarbonylpyrazolo[1,5-a]pyrimidin-7-one,
- 2-(2'-furyl)-6-methylpyrazolo[1,5-a]pyrimidin-7-one,
- 2-(2'-thienyl)-6-methylpyrazolo[1,5-a]pyrimidin-7-one,
- 3-ethoxycarbonyl-6-methylpyrazolo[1,5-a]pyrimidin-7-one,
- 2-methyl-5-methoxymethylpyrazolo[1,5-a]pyrimidin-7-one,
- 2-tert-butyl-5-trifluoromethylpyrazolo[1,5-a]pyrimidin-7-one,
- pyrazolo[1,5-a]pyrimidin-5-one,
- 2-methylpyrazolo[1,5-a]pyrimidin-5-one,
- 2,7-dimethylpyrazolo[1,5-a]pyrimidin-5-one,
- 2-phenylpyrazolo[1,5-a]pyrimidin-5-one,
- 2-ethylthiopyrazolo[1,5-a]pyrimidin-5-one,
- 2-carboxypyrazolo[1,5-a]pyrimidin-5-one,
- 7-aminopyrazolo[1,5-a]pyrimidin-5-one,
- 7-amino-2-methylpyrazolo[1,5-a]pyrimidin-5-one,
- 7-amino-2-phenylpyrazolo[1,5-a]pyrimidin-5-one,
- 7-amino-2-ethylthiopyrazolo[1,5-a]pyrimidin-5-one,
- 7-amino-2-carboxypyrazolo[1,5-a]pyrimidin-5-one,
and the addition salts thereof with an acid.

15. Composition according to any one of the preceding claims, characterized in that the addition salts with an acid for the compounds of formula (I) are chosen from the hydrochlorides, hydrobromides, sulphates, tartrates, benzenesulphonates, lactates, tosylates and acetates.

16. Composition according to any one of the preceding claims, characterized in that the compound(s) of formula (I) represent (s) from 0.0005 to 12% by weight relative to the total weight of the dye composition.

17. Composition according to Claim 16, characterized in that the compound(s) of formula (I) represent(s) from 0.0005 to 12% by weight relative to the total weight of the dye composition.

18. Composition according to any one of the preceding claims, characterized in that the oxidation base(s) is(are) chosen from para-phenylenediamines, bis(phenyl)alkylenediamines, para-aminophenols, orthoaminophenols and heterocyclic bases, and the addition salts thereof with an acid.

19. Composition according to any one of the preceding claims, characterized in that the oxidation base(s) represent(s) from 0.0005 to 12% by weight approximately relative to the total weight of the dye composition.

20. Composition according to Claim 19, characterized in that the oxidation base(s) represent(s) from 0.0005 to 6% by weight approximately relative to the total weight of the dye composition.

21. Composition according to any one of the preceding claims, characterized in that it also contains one or more additional couplers other than the compounds of formula (I) and/or one or more direct dyes.

22. Composition according to any one of the preceding claims, characterized in that the medium which is suitable for dyeing (or the support) consists of water or of a mixture of water and at least one organic solvent chosen from C₁-C₄ lower alcohols, glycerol, glycols and glycol ethers, aromatic alcohols, similar products and mixtures thereof.

23. Composition according to any one of the preceding claims, characterized in that it has a pH of between 3 and 12.

24. Composition according to any one of the preceding claims, characterized in that it is in the form of liquids, creams or gels or is in any other form which is suitable for dyeing keratin fibres, and in particular human hair.

25. Use of the compounds of formula (I) or the addition salts thereof with an acid, as defined in any one of Claims 1 to 15, as couplers in compositions for the oxidation dyeing of keratin fibres, and in particular human keratin fibres such as the hair, in combination with at least one oxidation base.

26. Process for the oxidation dyeing of keratin fibres, and in particular human keratin fibres such as the hair, characterized in that at least one dye composition as defined in any one of Claims 1 to 24 is applied to these fibres, the colour being developed at acidic, neutral or alkaline pH using an oxidizing agent which is added to the dye composition only at the moment of use, or which is present in an oxidizing composition that is applied simultaneously or sequentially in a separate manner.

27. Process according to Claim 26, characterized in that the oxidizing agent is chosen from hydrogen peroxide, urea peroxide, alkali metal bromates and persalts such as perborates and persulphates.

28. Multi-compartment dyeing "kit" or multi-compartment devices, in which a first compartment contains a dye composition as defined in any one of Claims 1 to 24, and a second compartment contains an oxidizing composition.
